# EUROPEAN PATENT APPLICATION

(11) **EP 3 858 969 A1**
(43) Date of publication of application: **04.08.2021**
(21) Application number: 20155118.1
(22) Date of filing: 03.02.2020
(51) Int. Cl.: C12M 1/06, C12M 1/00

(54) **PRETREATMENT ARRANGEMENT COMPRISING A SCRAPING DEVICE**

(71) Applicant: Sekab E-Technology AB, 891 26 Örnsköldsvik (SE)
(72) Inventor: SJÖBLOM, Anders, 891 78 BONÄSSUND (SE); HÄGGLUND, Karin, 892 92 DOMSJÖ (SE); SUNDVALL, Elias, 892 32 DOMSJÖ (SE)
(74) Representative: Kransell & Wennborg KB

(57) **Abstract**

The present disclosure generally relates to a pretreatment arrangement (100) for pretreatment of lignocellulosic biomass comprising a reactor vessel (101) extending along a longitudinal center line (102) and having an upstream inlet (103) for receiving biomass and a downstream outlet (104) for discharging biomass. The pretreatment arrangement (100) further comprising a scraping device (108) configured to scrape the interior walls (111) of the reactor vessel (101) and prevent the formation and build-up of deposits.

## Description

### TECHNICAL FIELD

The present disclosure generally relates to a pretreatment arrangement for pretreatment of lignocellulosic biomass comprising a reactor vessel extending along a longitudinal center line and having an upstream inlet for receiving biomass and a downstream outlet for discharging biomass. The pretreatment arrangement further comprises a scraping device. The present disclosure also relates to a system for treatment of lignocellulosic biomass comprising the pretreatment arrangement.

### BACKGROUND

Lignocellulosic residues from forestry are attractive as feedstocks for the production of renewable chemicals and fuels, since they are abundant, relatively inexpensive, and are not used for food. Lignocellulose consists mainly of lignin and two classes of polysaccharides, cellulose and hemicellulose. The polysaccharides can be hydrolyzed to sugars and converted to various fermentation products, e.g. bio-alcohols, by means of microorganisms, such as baker's yeast *Saccharomyces cerevisiae.*

The hydrolysis of cellulose is typically preceded by a pretreatment process, in which the hemicellulose is degraded, and the cellulose is made increasingly accessible to cellulolytic enzymes. During hydrolysis, the cellulose present is partly converted into glucose.

The pretreatment process is considered to be one of the more critical parts in the process of converting biomass into fermentation products, mainly because it affects downstream processes and strongly impacts the overall sugar yields. A variety of pretreatment processes exist, many of which rely on elevated temperatures and pressures, generally including catalytic agents.

The pretreatment process is typically carried out in a pretreatment arrangement, e.g. a pretreatment reactor. Such a pretreatment reactor generally comprises an inlet for receiving the biomass to be pretreated and an outlet for discharging the pretreated biomass, and a closed vessel wherein the pretreatment process is carried out.

One problem that may occur during pretreatment is that the dissolved, suspended or partially suspended biomass may deposit onto the interior walls of the reactor. The formation of deposits may be the result of charring of the biomass and burning of sugar and lignin. Such deposits may gradually increase, and eventually reduce the interior volume of the reactor vessel. This may result in a reduced efficiency of the reactor and the pretreatment process, which may then operate at decreased capacity, or be shut down, resulting in loss of revenue and increased production costs.

There is therefore a need for improvements with respect to methods to overcome problems with deposit formation during pretreatment of lignocellulosic biomass. Particularly, there is a need to provide a pretreatment system that is operationally stable and reliable and that allows for a stable and improved pretreatment process to be carried out.

### SUMMARY

In view of the above, it is an object of the present disclosure to provide improvements with respect to pretreatment systems, particularly with respect to reducing the formation of deposits within the reactor, and to secure an optimal flow of biomass within the reactor.

According to a first aspect of the present disclosure, there is provided a pretreatment arrangement for pretreatment of lignocellulosic biomass comprising a reactor vessel extending along a longitudinal center line and having an upstream inlet for receiving biomass and a downstream outlet for discharging biomass; the reactor vessel comprising an upper portion and a lower portion, wherein the arrangement further comprises a scraping device: the scraping device comprising a shaft and at least two scraping blades extending from the shaft; wherein the scraping device is configured to rotate about the longitudinal center line, wherein the scraping blades are configured to follow the contour of, without contacting the interior walls of at least a portion of the lower portion of the reactor vessel and wherein the shaft does not extend into the lower portion of the reactor vessel.

The present inventive concept is based on the realization that the scraping device secures a continuous flow of biomass in the vertical reactor, while scraping deposits formed on the interior walls of the reactor vessel. The pretreatment arrangement of the present disclosure secures that that there is no build-up of deposit inside the reactor, and the full interior volume of the reactor vessel can therefore be utilized for the pretreatment of lignocellulosic biomass.

The scraping device is adapted to scrape, but not to agitate or "whisk" the biomass flowing within the reactor. The scraping device comprises a shaft and at least two scraping blades extending therefrom. The scraping blades are configured to follow the contour of, without contacting the interior walls of at least a portion of the lower portion of the reactor vessel. The lower portion of the reactor vessel may correspond to the liquid (or slurry) phase within the reactor vessel during operation. A scraping function is thus achieved throughout the entire slurry containing part of the reactor vessel.

The fact that the shaft does not extend into the lower portion of the reactor vessel is advantageous as the shaft could otherwise form a surface for deposits to form and to build up against. The scraping device lacks a shaft in the portions of the reactor below the biomass slurry level.

In embodiments, the upper portion has a longitudinal extension corresponding to 10-50 % of the maximum longitudinal extension of the reactor vessel and the lower portion has a longitudinal extension corresponding to 50-90 % of the maximum longitudinal extension of the reactor vessel.

As mentioned, the upper portion of the reactor vessel corresponds to the portion of the reactor vessel arranged above the biomass slurry level within the reactor vessel during operation. The lower portion of the reactor vessel corresponds to the portion(s) of the reactor vessel arranged below the biomass slurry level within the reactor vessel during operation.

In embodiments, the scraping device is configured to provide a substantially laminar flow of biomass in the reactor vessel.

In other words, the biomass flows in a substantially continuous manner through the reactor vessel without yielding any significant turbulence within the reactor vessel or any upward flow of materials against the interior walls of the reactor vessel. The scraping device is thus adapted to scrape the walls, not to agitate or mix the biomass flowing within the vessel.

In embodiments, the scraping blades are configured to follow the contour of, without contacting the interior walls of substantially the entire lower portion of the reactor vessel.

The scraping device, and particularly the scraping blades have a shape in the longitudinal extension that corresponds to that of the reactor vessel. An improved scraping in the reactor vessel at the surfaces at risk for deposit formation; i.e. below the slurry level on the interior walls and in the lower portion of the reactor, is thereby achieved.

In embodiments, the sidewalls of at least a portion of the lower portion of the reactor vessel taper towards the outlet of the reactor vessel.

Hence, an improved discharge of pretreated biomass is achieved, and biomass is prevented from settling at the bottom surface of the reactor vessel.

The scraping blades are preferably arranged to provide an efficient scraping of the interior reactor walls without risking that these become damaged by the blades. Therefore, a small gap should be provided between the scraping blades and the interior walls.

In embodiments, the scraping blades are arranged at a distance, d1, from the interior walls of the reactor vessel, wherein the distance, d1, corresponds to from 0.5 to 20 %, preferably from 2 to 10 % of the inner diameter of the reactor vessel.

In embodiments, the shaft comprises a first end portion and a second end portion, and wherein each of the at least two scraping blades comprises a first end attached to the second end portion of the shaft and a second end attached to a blade connecting means, wherein the blade connecting means is arranged to follow the contour of at least a portion of the bottom surface of the reactor vessel.

The blade connecting means serves to connect and engage the scraping blades in the lower portion of the reactor vessel, and may be arranged above the outlet of the reactor vessel.

Depending on the size and shape of the reactor, the number of scraping blades may vary.

To secure an efficient scraping, the scraping device may comprise from 3 to 12 scraping blades, preferably from 3 to 8 scraping blades.

In embodiments, at least one of the scraping blades comprises an exterior surface facing the interior walls of at least a portion of the lower portion of the reactor vessel, and an interior surface facing the interior of the reactor vessel, and at least one blade surface extending at an angle, α2, from the interior surface to the exterior surface.

The blade surface is adapted to contact and peel off the deposit material formed on the interior walls of the reactor vessel.

Preferably, the blade surface extends at an angle, α2, of from 20 to 70 degrees, preferably from 30 to 60 degrees from the interior surface to the exterior surface.

This way, a relatively sharp blade surface is formed which is adapted to improve the scraping of the deposit material formed in the reactor. A blade surface extending at a sharper angle, α2, may efficiently scrape deposits that may have become firmly adhered to the interior walls of the reactor vessel. Such firmly adhered deposits may e.g. form due to biomass and sugars becoming charred or burned when the reactor vessel is operated at high temperatures and high pressures. This construction is also beneficial as the scraping blades follow the contour of the interior walls in a smooth manner preventing a turbulent flow of biomass against the interior walls. A turbulent flow, which is typically achieved with reactor agitators or whisks, is undesired in a pretreatment arrangement of the present disclosure. The construction of the scraping blades improves the laminar flow of biomass against the interior walls and within the reactor vessel.

In embodiments, the width, w1, of the interior surface of the at least one scraping blade corresponds to from 20 to 70%, preferably from 40 to 60% of the width, w2, of the exterior surface of the scraping blade(s).

Accordingly, the width of the exterior surface is larger than that of the interior surface such that the blade surface tapers towards the interior surface, and provides an angled or sloped surface intended to cut through any deposit material built up at the reactor walls.

In embodiments, the pretreatment arrangement of the present disclosure is adapted for steam explosion.

Steam explosion is an energy and cost-efficient pretreatment method for pretreatment of lignocellulosic biomass. During steam explosion, hot steam is used to increase the pressure in the reactor and to disrupt of bonding between polymeric components of the lignocellulosic biomass. Thereafter, decompression from an increased pressure to atmospheric pressure occurs, which results in that the lignocellulose biomass is broken up into smaller particle sizes.

In many pretreatment processes, such as during steam explosion, gases and volatile compounds contained in the lignocellulosic materials, are liberated when the biomass is degraded or partially degraded. The liberation of gases may also be the result of chemical reactions occurring in the reactor. Such liberated gases may accumulate in the upper portion of the reactor vessel and cause undesired fluctuations in the pressure and temperature in the reactor. The stability of the pretreatment process may thereby be impaired, which may contribute to the problem of deposit formation within the reactor, and particularly on the interior walls thereof.

Hence, in embodiments, the pretreatment arrangement further comprises a gas valve configured to remove gas from the reactor vessel.

In embodiments, the pretreatment arrangement comprises a sluice vessel arranged downstream of and in fluid communication with the reactor vessel, wherein the sluice vessel is configured to discharge biomass received from the outlet of the reactor vessel.

The sluice vessel secures an improved discharge of pretreated biomass from the reactor vessel, and is particularly advantageous for pretreatment processes involving steam explosion. If the pretreatment involves steam explosion, such a process poses demands on the equipment utilized. The high temperatures and pressures used within the reactor may result in the formation of deposits within the reactor, and such deposits may build up on the reactor walls. The provision of a sluice vessel allows the biomass to first be treated in the reaction vessel under optimum conditions for pretreatment (i.e. suitable time, pressure and temperature), and subsequently, the pressure may be increased within the sluice vessel; i.e. outside of the reactor vessel. Upon discharge from the sluice vessel, the pressure drops and results in division of the material into smaller particles. Accordingly, the "harsh" process conditions associated with large pressure variations, are performed separate from the reactor vessel, and the burning or charring of sugars and biomass within the reactor vessel, can be avoided. The discharge of biomass is therefore performed in a controlled and improved manner.

According to another aspect, there is provided a system for treatment of lignocellulosic biomass comprising a pretreatment arrangement as described hereinbefore and a hydrolysis unit arranged in fluid communication with and downstream of the pretreatment arrangement, and optionally, a fermentation unit arranged in fluid communication with and downstream of the hydrolysis unit.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 illustrates a pretreatment arrangement according to an exemplary embodiment of the present disclosure.
Figure 2a illustrates a perspective view of a scraping device according to an exemplary embodiment of the present disclosure.
Figure 2b illustrates a side view of a scraping device according to an exemplary embodiment of the present disclosure.
Figure 2c is a zoomed in view of the scraping device of figure 2b, illustrating the blade connecting means and scraping blades extending from the blade connecting means.
Figure 2d is a zoomed in view of the scraping blade according to an exemplary embodiment of the scraping device of the present disclosure.
Figure 3 schematically illustrates a system for treatment of lignocellulosic biomass according to the present disclosure.

### DETAILED DESCRIPTION

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

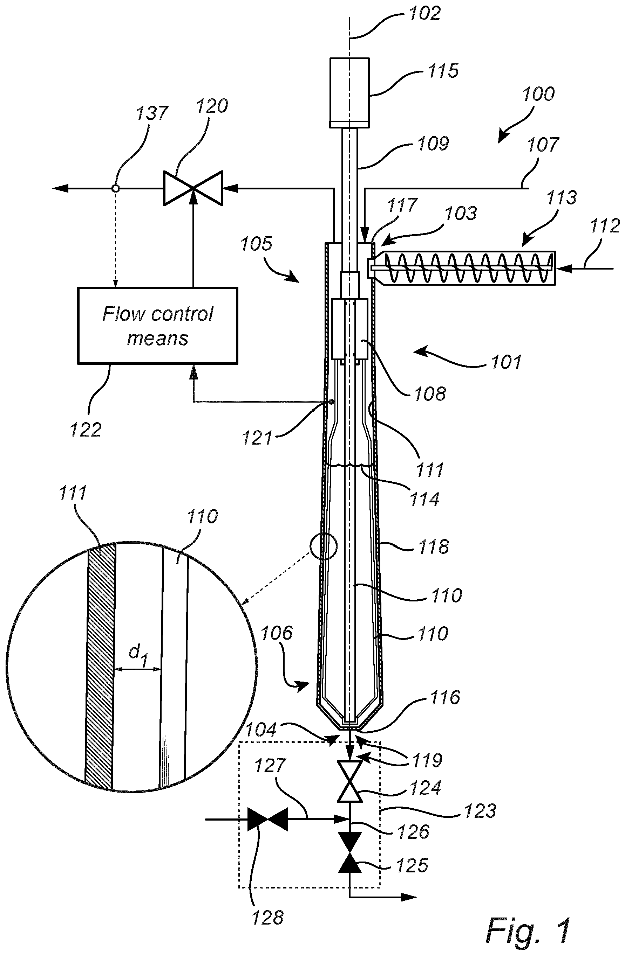

Figure 1 illustrates a pretreatment arrangement 100 for pretreatment of lignocellulosic biomass comprising a reactor vessel 101 extending along a longitudinal center line 102 and having an upstream inlet 103 for receiving biomass and a downstream outlet 104 for discharging biomass; the reactor vessel comprising an upper portion 105 and a lower portion 106, wherein the arrangement 100 further comprises a scraping device 108: the scraping device 108 comprising a shaft 109 and at least two scraping blades 110 extending from the shaft 109; wherein the scraping device 108 is configured to rotate about the longitudinal center line 102, and wherein the scraping blades 110 are configured to follow the contour of, without contacting the interior walls 111 of at least a portion of the lower portion 106 of the reactor vessel 101 and wherein the shaft 109 does not extend into the lower portion 106 of the reactor vessel 101.

Lignocellulosic biomass 112 enters the reactor vessel 101 by means of the inlet 103. The inlet is arranged in the upper portion 105 of the reactor vessel. In figure 1, the biomass 112 is fed into the reactor vessel 101 by means of a plug screw feeder 113. The plug screw feeder 113 secures an even flow of biomass into the reactor vessel 101. The pretreatment arrangement is not limited to a specific type of inlet or feeding means, but any inlet or means for feeding biomass, known to those skilled in the art, may be used.

The lignocellulosic biomass may be, but is not limited to, hardwoods, softwoods, sugarcane bagasse, energy cane, corn stover, corn cobs, corn fibers, straw from rice, wheat, rye and other crops residues.

The scraping blades 110 are arranged to follow the contour of, without contacting the interior walls 111 of the reactor vessel 101. In operation, the scraping device 108 rotates about the longitudinal center line 102 in close proximity of the interior walls 111 of the reactor vessel 101 in order to prevent biomass from depositing on the interior walls 111 and to remove deposits already formed. The scraping blades 110 should not contact the interior walls 111, but be arranged at a distance from the walls in order to prevent the scraping blades 110 from scratching or damaging the interior walls 111 of the reactor vessel 101.

The scraping blades 110 are arranged to extend from a shaft 109 of the scraping device 108. The shaft may be arranged partially outside the reactor vessel 101, and, as illustrated in figure 1, may be arranged to extend into the reactor vessel 101. Alternatively, the shaft may be arranged completely outside the reactor vessel. However, the shaft 109 should not be arranged in a portion of the reactor vessel 101 below the slurry level 114 since the shaft 109 may then form another surface onto which deposits may form and build up within the reactor vessel 101. Instead, the shaft 109 may be configured to extend in an upper portion 105 of the reactor vessel 101, above the slurry level. The pretreatment process is thereby improved since the entire volume of the reactor vessel 101 may be utilized, and the process does not need to be halted or shut down due to deposits building up in the reactor vessel 101.

The upper portion 105 of the reactor vessel 101 has a longitudinal extension corresponding to 10-50 % of the maximum longitudinal extension of the reactor vessel 101, and the lower portion 106 has a longitudinal extension corresponding to 50-90 % of the maximum longitudinal extension of the reactor vessel 101.

The upper portion 105 of the reactor vessel 101 corresponds to the portion of the reactor vessel 101 arranged above the biomass slurry level within the reactor vessel 101 during operation.

The lower portion 106 of the reactor vessel 101 corresponds to the portion of the reactor vessel 101 arranged below the biomass slurry level within the reactor vessel 101 during operation.

In other words, the interface between the upper 105 and the lower portion 106 corresponds to the maximum biomass slurry level during operation.

In embodiments, the scraping device 100 is configured to provide a substantially laminar flow of biomass in the reactor vessel 101.

Accordingly, the biomass flows in a substantially continuous manner at a low velocity from the inlet 103 to the outlet 104 of the reactor vessel 101. The purpose of the scraping device 108 is to scrape, not to agitate or "whisk" the biomass within the reactor. A "substantially laminar flow", as used herein, is a flow which is predominantly laminar and does not cause significant agitation, vigorous mixing or an upward flow of biomass in the reactor vessel. The substantially laminar flow may also be referred to as a plug flow.

The scraping device 108 may be configured to rotate about the longitudinal center line 102 at a speed of from 1 to 30 rpm, e.g. from 1 to 15 rpm. It should however be noted that the rotational speed will vary depending on the size of the reactor, the biomass to be treated etc. The rotational speed will naturally be different if the reactor vessel is larger and if the biomass to be pretreated e.g. has a higher viscosity.

The scraping device 108 may comprise means 115 for controlling the rotational speed. Such means 115 may comprise a motor adapted to regulate the speed of the scraping device 108.

The scraping blades 110 are preferably configured to follow the contour of, without contacting the interior walls 111 of substantially the entire lower portion 106 of the reactor vessel 101.

Accordingly, the scraping blades 110 are adapted to scrape the surfaces at the highest risk for deposit formation.

The reactor vessel 101 is not limited to a particular shape or size, but may be adapted depending on the pretreatment to be carried out and the scale of such a pretreatment process. The pretreatment arrangement may be used in both large-scale and small-scale pretreatment processes.

Typically, the reactor vessel 101 is a vertical reactor vessel as illustrated in figure 1.

Typically, the reactor vessel 101 is cylindrical and has a circular or oval cross-section.

In embodiments, the reactor vessel 101 has a rotational symmetry with respect to the longitudinal center line 102.

In embodiments, the reactor vessel 101 is defined by a bottom surface 116 and a top surface 117 and side walls 118 extending between the bottom surface 116 and the top surface 117.

The sidewalls 118 of at least a portion of the lower portion 106 may taper towards the outlet 104 of the reactor vessel 101.

In other words, the side walls 118 taper towards the bottom surface 116 of the reactor vessel 101.

This is to facilitate and improve the discharge of pretreated biomass 119 from the outlet 104 of the reactor vessel 101.

In embodiments, the scraping blades are arranged at a distance, d1, from the interior walls 111 of the reactor vessel 101, wherein the distance, d1, corresponds to from 0.3 to 20 %, from 0.5 to 20 %, from 1 to 20%, or preferably from 2 to 10% of the inner diameter of the reactor vessel 101.

This distance, d1, forms a gap between the outermost surface of the scraping blades 110 and the interior walls 111 of the reactor vessel 101. This is to ensure that deposits formed on the interior walls 111 are scraped off and to ensure that the scraping blades do not damage or scrape the interior walls 111 of the reactor vessel 101.

For example, if the reactor vessel 101 has an inner diameter in the range of from 200 to 800 mm, the distance, d1, between the outermost surface of the scraping blades 110 and the interior walls 111 of the reactor vessel 101 may e.g. be from 1 to 30 mm, such as from 5 to 13 mm. As already mentioned, the distance, d1, will depend on the size of the reactor vessel 101.

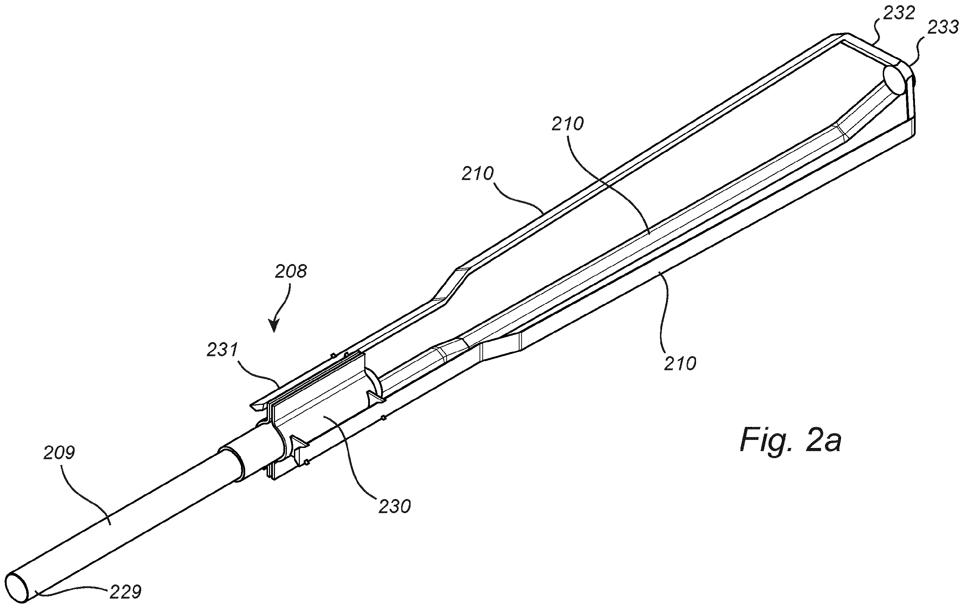

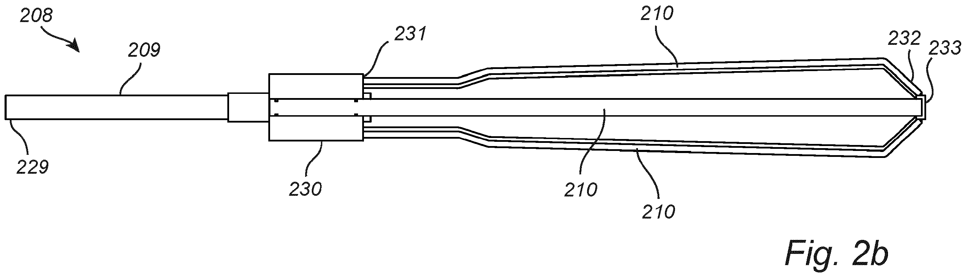

Figures 2a and 2b illustrate exemplary embodiments of a scraping device 208 for use in a pretreatment arrangement of the present disclosure. As illustrated in figures 2a and 2b, the shaft 209 comprises a first end portion 229 and a second end portion 230, and wherein each of the at least two scraping blades 210 comprises a first end 231 attached to the second end portion 230 of the shaft 209 and a second end 232 attached to a blade connecting means 233, wherein the blade connecting means 233 is arranged to follow the contour of at least a portion of the bottom surface of the reactor vessel.

The first end portion 229 of the shaft 209 may be arranged partially outside of the reactor vessel. The second portion 230 of the shaft 209 may be arranged partially inside of the reactor vessel (but not below the slurry level).

The blade connecting means 233 is arranged in the lower portion of the reactor vessel. The blade connecting means 233 may be a plate, disc, ring or a bearing which serves to connect and engage the second ends 232 of the scraping blades 210 in a lower portion of the reactor vessel. The blade connecting means 233 may have any shape and size. In embodiments, the blade connecting means 233 is circular.

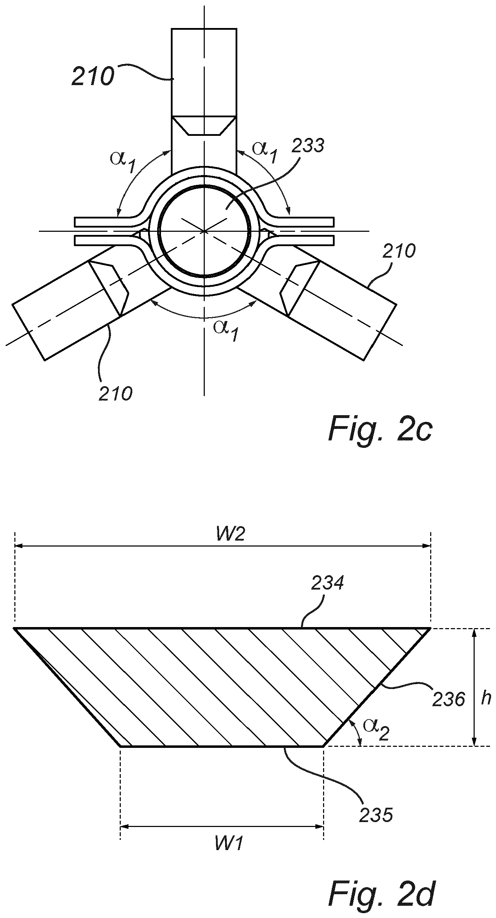

Figure 2c illustrates a blade connecting means 233, in the form of a circular bottom plate and scraping blades 210 extending from the bottom plate 233. In embodiments, the angle, α1, between the scraping blades 210 is between 60° and 120°. The angle, α1, between the scraping blades 210, is dependent on the number of scraping blades 210, and it is possible, but not necessary that the angle, α1, is the same between the scraping blades 210. In figure 2c, the angle, α1, between the scraping blades 210 is the same, namely 120°. A circumferential symmetry in the arrangement of scraping blades 210 may be beneficial from an operational as well as a production point of view.

The scraping device 208 illustrated in figures 2a-c comprises three scraping blades 210. It should however be noted that the scraping device 208 is by no means limited to a specific number of scraping blades 210. The number of scraping blades 210 is dependent on the size of the blades 210 and on the size of the reactor vessel. At least two scraping blades 210 are necessary to achieve a sufficient scraping function. The number of scraping blades 210 should not be too large, since the scraping blades may then form additional surfaces onto which biomass deposits may settle in the reactor vessel.

In embodiments, the scraping device 208 comprises from 2 to 12 scraping blades, such as from 3 to 12 scraping blades, such as from 2 to 8 scraping blades, preferably from 3 to 12 scraping blades, or 3 to 8 scraping blades.

This is beneficial to achieve an improved scraping of the interior reactor walls and to prevent the build-up of deposits on the walls and on the scraping device. It is also beneficial from a production point of view.

As best illustrated in figure 2d, at least one of the scraping blades 210 comprises an exterior surface 234 facing and following the contour of the interior walls of at least the lower portion of the reactor vessel, and an interior surface 235 facing the interior of the reactor vessel, and at least one blade surface 236 extending at an angle, α2, from the interior surface 235 to the exterior surface 234.

To enable an improved scraping and to prevent a turbulent flow around the interior walls of the reactor vessel, the blade surface 236 may extend at an angle, α2, of from 20 to 70 degrees, preferably from 30 and 60 degrees from the interior surface 235 to the exterior surface 234.

The blade surface 236 thus provides a sharp knife surface which may efficiently scrape off biomass that may have become stuck to the interior walls or is about to get stuck on the interior walls. This construction is also beneficial as the scraping blades 210 follow the contour of the interior walls in a smooth manner preventing a turbulent flow of biomass against the interior walls. A turbulent flow, which is typically achieved with reactor agitators or whisks, is undesired in a pretreatment arrangement of the present disclosure. The construction of the scraping blades secures a substantially laminar flow of biomass against the interior walls and within the reactor vessel.

In embodiments, the width, w1, of the interior surface 235 of the at least one scraping blade 210 is smaller than the width, w2, of the exterior surface 234 of the scraping blade 210. In other words, the blade surface 236 tapers towards the interior surface.

For example, the width, w1 of the interior surface 235 may correspond to from 20 to 70%, preferably from 40 to 60% of the width, w2 of the exterior surface 234 of the scraping blade 210.

The widths, w1 and w2, are dependent on the size of the reactor vessel and may vary significantly if the reactor vessel is a small reactor for small-scale pretreatment processes or if it is a large reactor used for large-scale production.

As an illustrative, but non-limiting example, for a reactor vessel having a diameter of about 200 mm, the width, w2, of the exterior surface 234 may be from 10 to 60 mm, e.g. from 30 to 40 mm. The width, w1, of the interior surface 235 may e.g. be from 4 to 42 mm, e.g. from 12 to 22 mm.

The exterior surface 234 of the scraping blade 210 may be generally flat or curvilinear. This surface is configured to face the interior walls of the reactor vessel. In the event that the scraping blade(s) 210 would contact the reactor walls, the generally flat or curvilinear surface reduces the risk of cutting or scraping the reactor walls.

The pretreatment arrangement of the present disclosure is not limited to a particular type of pretreatment process, but any process used to pretreat and render the biomass more susceptible to hydrolytic enzymes may be used.

In embodiments, the pretreatment arrangement of the present disclosure is adapted for steam explosion. The steam explosion may be non-catalyzed or acid catalyzed.

During steam explosion, hot steam is used to increase the pressure in the reactor and to disrupt of bonding between polymeric components of the lignocellulosic biomass. Thereafter, decompression from an increase of pressure to atmospheric pressure occurs, which results in that the lignocellulose biomass is broken up into smaller particle sizes. The disruption of bonds between polymeric materials during steam explosion may be catalyzed by hot water on its own, components such as acetic acid that is released during the degradation of hemicellulose, or by addition of acids in liquid or gaseous form, such as sulfur dioxide or sulfuric acid.

In figure 1, the addition of steam into the reactor vessel 101 is denoted 107.

With reference to figure 1, the pretreatment arrangement 100 may further comprise a gas valve 120 configured to remove gas from the reactor vessel 101. The gas valve 120 preferably has an adjustable opening configuration. The gas valve 120 may be attached to the reactor vessel 101 or connected to the reactor vessel 101 by means of a tube (the latter of which is illustrated in figure 1).

The reactor vessel 101 may further comprise measuring means 121 for measuring a number of process parameters of the pretreatment in the reactor vessel 101. Such process parameters include at least a temperature parameter and a pressure parameter.

The pretreatment arrangement 100 may further comprise gas flow control means 122 configured to adjust the outflow of gas from the gas valve 120 in response to the measured process parameters. This way, a controlled outflow of gas from the reactor vessel 101 is achieved. Accordingly, a more controlled pretreatment is achieved. The temperature and pressure have been identified as key parameters, together sufficient for achieving stable pretreatment conditions, which implies an efficient pretreatment and reduced formation of deposits on the interior walls of the reactor.

The relationship between the pressure and the temperature is preferably monitored throughout the pretreatment reaction, and when the temperature (or the pressure) deviates from a desired, preferably substantially constant, pressure-to-temperature relationship, this is typically an indication that gases, e.g. inert gases, have started to accumulate within the reactor vessel 101. Such gases may then be removed from the reactor vessel 101 by means of the gas valve 120. The outflow of gas from the gas valve 120 may be adjusted and regulated in response to deviations in temperature or pressure.

In embodiments, the gas flow control means 122 is configured to adjust the outflow of gas from the gas valve 120 in response to the relationship between the temperature and pressure, e.g. expressed as a ratio between temperature and pressure, so as to achieve a controlled flow of gas out from the reactor vessel.

Furthermore, the gas flow control means 122 may be configured to determine a ratio between the temperature parameter and the pressure parameter and to adjust the outflow of gas from the gas valve 120 in response to the determined ratio. The gas flow control means 122 may be configured to adjust the outflow of gas from the gas valve 120 if the determined ratio deviates from a predetermined reference ratio interval for the pretreatment.

By adjusting the outflow of gas from the gas valve 120 in response to the relationship between the temperature and pressure, the temperature and pressure, or the ratio between temperature and pressure, can be held within a predetermined interval of deviation (basically constant, if the interval is comparatively narrow) for the specific pretreatment to be carried out.

Such pretreatment arrangements will counteract or compensate for imbalance between the temperature and pressure within the reactor caused by the liberation of gases from the biomass during degradation or partial degradation, and which is particularly problematic if the pretreatment is carried out by applying steam or additional catalysts, particularly gaseous catalysts, leading to an excess amount of accumulated gases in the reactor.

In embodiments, the pretreatment arrangement 100 comprises a flow meter 137 configured to measure the outflow of gas from the reactor vessel 101. The flow meter 137 may indicate that the flow of gas is too high or too low, and the gas flow control means 122 may be configured to adjust the opening of the gas valve 120 in response to the measured outflow of gas.

In embodiments, the pretreatment arrangement 100 comprises a sluice vessel 123 arranged downstream of and in fluid communication with the reactor vessel 101, wherein the sluice vessel 123 is configured to discharge biomass received from the outlet 104 of the reactor vessel 101. A sluice vessel 123 is advantageous for controlling the discharge of the pretreated lignocellulosic material 119 and for improving the control of the process conditions within the reactor vessel 101.

The sluice vessel 123 may comprise a first discharge valve 124, a second discharge valve 125 arranged downstream of the first discharge valve 124 and a compartment 126 arranged between the first 124 and the second 125 discharge valves. The sluice vessel 123 may further comprise means 127 for increasing the pressure in the compartment 126 of the sluice vessel 123. The means 127 for increasing the pressure may be a tube adapted to supply gas, e.g. steam to the compartment 126, and may e.g. comprise a valve 128 to control the supply of gas.

The provision of a sluice vessel 123 downstream of the reactor vessel 101 allows the pressure to be remained within the reactor vessel 101, while the pressure may be significantly increased and decreased in the sluice vessel 123. A pressure drop resulting from the discharge of biomass from the second discharge valve 125 of the sluice vessel 123 improves the division of the pretreated biomass 119 into smaller particles. The sluice vessel 123 may therefore be adapted for steam explosion. Performing the steam explosion outside of and downstream of the reactor vessel 101 is beneficial in that potential deposits resulting from harsh pretreatment conditions (high pressures and high temperatures) are prevented within the reactor vessel 101. By increasing the pressure inside the sluice vessel 123, a higher pressure drop is obtained when the biomass is discharged from the second discharge valve 125. As a result, the treated biomass will be divided into smaller pieces compared to if a direct discharge from the reactor vessel 101 is performed.

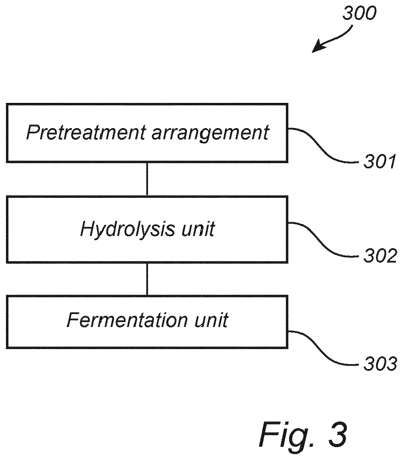

With reference to figure 3, the present disclosure further provides a system 300 for treatment of lignocellulosic biomass comprising a pretreatment arrangement 301 for pretreatment of lignocellulosic biomass according to the first aspect of the present disclosure, a hydrolysis unit 302 arranged downstream of and in fluid communication with the pretreatment arrangement 301, and optionally, a fermentation unit 303, such as a fermentation vessel, arranged downstream of and in fluid communication with the hydrolysis unit 302. The system 300 may comprise additional units and components known to those skilled in the art. For example, a separation unit may be arranged between the pretreatment arrangement 301 and the hydrolysis unit 302, and/or between the hydrolysis unit 302 and the fermentation unit 303.

In the hydrolysis unit, the pretreated biomass is subject to enzymatic hydrolysis by means of saccharification enzymes. Fermentation of the hydrolysate into a target chemical is typically performed by means of fermenting organism, such as bacteria and/or yeast. The system 300 may also comprise a product recovery unit, such as distillation or ion exchange chromatography, arranged downstream of and in fluid communication with the fermentation unit 303.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A pretreatment arrangement (100) for pretreatment of lignocellulosic biomass comprising a reactor vessel (101) extending along a longitudinal center line (102) and having an upstream inlet (103) for receiving biomass and a downstream outlet (104) for discharging biomass; said reactor vessel (101) comprising an upper portion (105) and a lower portion (106), wherein said pretreatment arrangement (100) further comprises a scraping device (108, 208): said scraping device (108, 208) comprising a shaft (109, 209) and at least two scraping blades (110, 210) extending from said shaft (109, 209); wherein said scraping device (108, 208) is configured to rotate about said longitudinal center line (102), **characterized in that** said scraping blades (110, 210) are configured to follow the contour of, without contacting the interior walls (111) of at least a portion of said lower portion of said reactor vessel (101) and **in that** said shaft (109, 209) does not extend into said lower portion (106) of said reactor vessel (101).

2. A pretreatment arrangement (100) according to claim 1, wherein said upper portion (105) has a longitudinal extension corresponding to 10-50 % of the maximum longitudinal extension of said reactor vessel (101) and said lower portion (106) has a longitudinal extension corresponding to 50-90 % of the maximum longitudinal extension of said reactor vessel (101).

3. A pretreatment arrangement (100) according to claim 1 or claim 2, wherein the scraping device (108) is configured to provide a substantially laminar flow of biomass in said reactor vessel (101).

4. A pretreatment arrangement (100) according to any one of the preceding claims, wherein said scraping blades (110, 210) are configured to follow the contour of, without contacting the interior walls (111) of substantially the entire lower portion (106) of said reactor vessel (101).

5. A pretreatment arrangement (100) according to any one of the preceding claims, wherein the sidewalls (118) of at least a portion of said lower portion (106) of said reactor vessel (101) taper towards the outlet (104) of said reactor vessel (101).

6. A pretreatment arrangement (100) according to any one of the preceding claims, wherein said scraping blades (110, 210) are arranged at a distance, d1, from said interior walls (111) of said reactor vessel (101), wherein said distance, d1, corresponds to from 0.5 to 20 %, preferably from 2 to 10 % of the diameter of said reactor vessel (101).

7. A pretreatment arrangement (100) according to any one of the preceding claims, wherein said shaft (109, 209) comprises a first end portion (229) and a second end portion (230), and wherein each of said at least two scraping blades (110, 210) comprises a first end (231) attached to said second end portion (230) of said shaft (109, 209) and a second end (232) attached to a blade connecting means (233), wherein said blade connecting means (233) is arranged to follow the contour of, without contacting at least a portion of the bottom surface (116) of said reactor vessel (101).

8. A pretreatment arrangement (100) according to any one of the preceding claims, wherein said scraping device (108) comprises from 3 to 12 scraping blades (110, 210), preferably from 3 to 8 scraping blades (110, 210).

9. A pretreatment arrangement (100) according to any one of the preceding claims, wherein at least one of said scraping blades (110, 210) comprises an exterior surface (234) facing and following the contour of the interior walls (111) of at least a portion of said lower portion (106) of said reactor vessel (101), and an interior surface (235) facing the interior of said reactor vessel (101), and at least one blade surface (236) extending at an angle, α2, from said interior surface (235) to said exterior surface (234).

10. A pretreatment arrangement (100) according to claim 9, wherein said blade surface (236) extends at an angle, α2, of from 20 to 70 degrees, preferably from 30 and 60 degrees from said interior surface (235) to said exterior surface (234).

11. A pretreatment arrangement (100) according to any one of claims 9 or 10, wherein the width, w1, of the interior surface (235) of said at least one scraping blade (110, 210) corresponds to from 20 to 70%, preferably from 40 to 60% of the width, w2, of said exterior surface (234) of said scraping blade (110, 210).

12. A pretreatment arrangement (100) according to any one of the preceding claims, wherein said pretreatment arrangement is adapted for steam explosion.

13. A pretreatment arrangement (100) according to any one of the preceding claims, further comprising a gas valve (120) configured to remove gas from said reactor vessel (101).

14. A pretreatment arrangement (100) according to any one of the preceding claims, wherein said pretreatment arrangement (100) comprises a sluice vessel (123) arranged downstream of and in fluid communication with said reactor vessel (101), wherein said sluice vessel (123) is configured to discharge biomass received from the outlet (104) of said reactor vessel (101).

15. A system (300) for treatment of lignocellulosic biomass comprising a pretreatment arrangement (301) according to any one of claims 1-14 and a hydrolysis unit (302) arranged in fluid communication with and downstream of said pretreatment arrangement (301), and optionally, a fermentation unit (303) arranged in fluid communication with and downstream of said hydrolysis unit (302).
